# DEMANDE DE BREVET EUROPEEN

(11) **EP 2 995 338 A1**
(43) Date de publication de la demande: **16.03.2016**
(21) Numéro de dépôt: 15382443.8
(22) Date de dépôt: 09.09.2015
(51) Int. Cl.: A61M 25/06, A61M 25/01, A61B 17/34, A61M 25/00

(54) **DISPOSITIF POUR PLACER UN CATHÉTER DANS UN POINT D'APPLICATION D'UN MÉDICAMENT SOUS CONTRÔLE DIRECT PAR ÉCHOGRAPHIE**

(30) Priorité: 12.09.2014 ES 201431321
(71) Demandeur: Sainz Lopez, Julio, 08230 Matadepera (ES)
(72) Inventeur: Sainz Lopez, Julio, 08230 Matadepera (ES)

(57) **Abrégé**

Un dispositif (1) pour placer un cathéter (3) dans un point d'application d'un médicament avec un contrôle direct au moyen de l'échographie qui comprend une aiguille (2) creuse courbe dans un matériau détectable par échographie (4), et un cathéter (3) aux formes et dimensions adéquates pour être introduit à l'intérieur de cette aiguille (2); où le cathéter (3) comprend une section tubulaire (5) pour l'introduction du médicament douée de quelques perforations latérales (6) et achevée par une extrémité fermée (7), et une prolongation filiforme (8) de manipulation, et des références de position par rapport à l'aiguille (2)

## Description

Dispositif pour placer un cathéter dans un point d'application d'un médicament avec un contrôle direct au moyen de l'écographie.

### OBJECTIF DE L'INVENTION

L' invention présente se rapporte à un dispositif pour placer un cathéter dans un point d'application d'un médicament, comme un anesthésique local pour le contrôle de la douleur aigüe ou chronique le long d'un nerf, avec un contrôle direct au moyen d'un ecôgraphe.

### ANTÉCÉDENTS DE L'INVENTION

Il existe un ample éventail de dispositifs (des aiguilles, des trocars) sur le marché depuis des années pour placer un cathéter le long d'un nerf, et administrer un anesthésique à travers du dit cathéter.

Les aiguilles creuses sanitaires permettent de passer à travers de sa lumière des guides et/ou des cathéters de différents matériaux (plastiques, métalliques etc.), ayant différentes les longueurs, les diamètres des lumières intérieures, les diamètres extérieurs, les géométrie de la pointe, de doubles triples ouvertures, etc. situés dans la pointe ou ailleurs dans sa longueur, les matériaux de construction, et des marquages extérieurs pour évaluer la profondeur d'insertion, mais toutes ces aiguilles sont droites et elles ne peuvent, pas avoir une utilisation comme il est attendu dans l'invention.

Également on connaît aussi des aiguilles solides d'une multitude de tailles et de formes, droites ou avec des courbes, avec pointes d'infinité de formes, de diamètres différents, mais aucune ne permet le passage d'autres matiéraux pour son placement, ce qui empêche également son utilisation comme il est attendu dans l'invention.

À l'égard des cathéters, ils sont de différents matériau, plus ou moins rigides, et une longueur variable; mais tous ont en commun que les ouvertures, où abouti la médication, sont situées dans la pointe ou tout près d'elle, ce qui empêche aussi son utilisation comme il est attendu dans l'invention.

Le placement des cathéters par le système traditionnel (jusqu'à présent utilisé) implique divers problèmes dérivés du manque de contrôle de la pointe du cathéter, parmi lesquels il faut énumérer :
1.- Le déplacement involontaire du cathéter qui est très fréquent (20 à 40 % des applications) et il augmente avec le passage des jours. Il se produit, ou bien au moment du retrait de l'aiguille introductive ou par la suite, avec la perte résultante d'effectivité, puisque la médication ne reste pas appliquée dans le lieu correct
2.-Impossible un replacement en cas de déplacement du cathéter, puisque nous n'avons pas de contrôle de la pointe après être introduit sous la peau ce qu'il oblige son retrait complet et à répéter toute la technique au complet depuis le début, autant de fois que nécessaire. Ce qui implique gène pour le patient et une augmentation du coût économique des matériaux et du temps employé.
3.-Si une rupture du cathéter survient (1 %), effet inhabituelle, quand elle se produit, le fragment qui reste retenu sous la peau, oblige à décider sur les deux options possibles : ou bien le laisser avec le risque que comporte laisser un corps étrange (infection, une migration en pouvant blesser des structures), ou bien chirurgicalement l'extraire que en fonction de leur emplacement implique un risque important de blesser des tissus pour le chercher, et même de, ne pas le trouver, donné son petit calibre et longueur du fragment. La décision à prendre parfois est complexe et coûteuse, tant dans des moyens matériels comme des séquelles physiques (une lésions internes, une cicatrice).

### DESCRIPTION DE L'INVENTION

Le dispositif de l'invention permet :
1.- Placer le cathéter avec précision, on faisant se rencontrer exactement les ouvertures du même dans le point d'application (typiquement a côté du nerf sélectionné), tout sous contrôle échographique. Cela produit l'application du médicament dans l'endroit parfait pour obtenir leurs résultats.
2.-En plus, permet la fixation des deux extrémités à la peau, en diminuant le risque de déplacement involontaire, tant au moment du placement comme a posteriori, tout en maintenant son effectivité pendant plus de temps, de façon a gérer avec présicion un médicament par moyen du dit cathéter.
3.-En plus, pendant son emplacement initial, ou par la suite, si s'est produit un déplacement de l' emplacement des ouvertures, ou si on a besoin de déplacer le cathéter pour atteindre une autre zone, ayant les deux côtés du cathéter contrôlés, permet de le déplacer dans les deux sens selon la nécessité et l 'emplacer à un nouveau emplacement, facilement, sans la nécessité de réaliser tout le procédé depuis le début.
4.-En plus, il est extrêmement avantageux qu'en cas d'une rupture du cathéter, les deux extrémités sont sur la peau, aucun fragment ne reste jamais sous la peau, c'est suffisant étirer simplement de ces deux extrémités.

Selon l'invention, le dispositif comprend une aiguille creuse courbe de matériau détectable au moyen de l' écographie, et un cathéter formel et d'une taille adaptée pour être introduit par la dite aiguille, la technique de placement étant réalisée sous une vision échographique, puisque l'écographie est capable d'affiicher la position de l'aiguille, du nerf et de la distribution parmi les tissus, du médicament administrée.

Dans ce document comme aiguille creuse et courbe on entend n'importe quelle aiguille creuse avec différente trajectoire de la droite, de façon à ce que sa pointe s'approche à son entrée, en pouvant aussi être en forme de U, V et similaires. De plus, cette aiguille peut extérieurement incorporer un marquage gradué pour permettre un contrôle visuel direct sur la profondeur de l'aiguille et du nerf. En outre, la pointe de l'aiguille possède de préférence son extrémité émoussée, étant entendu que émoussée vuet dire qui n'est pas très aiguisé pour abîmer les tissus sensibles ou fonctionnelles en cas de contact accidentel avec les mêmes, tout en restant suffisamment pointu pour permettre une progression facile à travers des tissus. L'acuité de la pointe est déterminé dans chaque cas, car il faut combiner ces deux variables.

Selon l'invention, le cathéter se compose : d'une partie tubulaire pour l'introduction du médicament, doué de quelques ouvertures latéraux pour aboutir du même, et achevé par une extrémité fermée; une prolongation filiforme de manipulation; et des références de position par rapport à l'aiguille. Les références de position du cathéter par rapport à l'aiguille seront utiler pour déterminer la position exacte des perforations latérales près du nerf ou la zone à laquelle on désire fournir le médicament - avec l'aide de l'aiguille et de l'écographie. L'écographie, comme il a pris rendez-vous, va permettre la réalisation de la technique sous une vision directe, avec une complète et effective réalisation.

On préférence, le prolongement filiforme est solide ou aveugle, et cette différence de la par rapport a la configuration de la section tubulaire creux, elle peut également être détectable par l'écographe, qui fourni une référence de position supplémentaire visible dans l'art de réaliser le placement précis du cathéter.

L'utilisation est la suivante: l'aiguille courbe fait possible pénétrer à travers la peau et des tissus et, suivant sa courbure et en passant à côté du nerf, de continuer dans la même direction jusqu'à ce qu'elle sorte à un autre point de la peau. Une fois que les deux extrémités sont en dehors de la peau, le cathéter est inséré a travers l'entrée de l'aiguille, qui lange l'intérieur jusqu'à ce que sa pointe apparaisse par le biais de la pointe de l'aiguille, moment ou est saisie cette pointe du cathéter et de commencer à extraire l'aiguille avec surveillance par l'écographie jusqu'à, ce que la pointe de celle-ci, élément détectable par l'ecographe, est situé de sorte que, avec les références de la position du cathéter, l'on puisse déterminer ensuite le placement des ouvertures, tout juste au point qui est le nerf, on tire alors de la pointe du cathéter au préalable saisie, ce qui rend que le cathéter s'introduit dans les tissus du patient. Avec les marques de position du cathéter - et donc de leurs ouvertures latéraux au regard de l'aiguille, et en sachant où la pointe de celle-ci est, déjà les dites ouvertures se trouvent près du nerf affecté; ensuite on fixe fermement la pointe de cathéter pour le reste pour qu'il ne soit pas déplacé, et finalement l'aiguille est retiré définitivement, le cathéter reste placé et l'on peut connecter la section tubulaire à la fourniture du médicament, généralement un anesthésique.

Avec cette simple technique, réalisable grâce au dispositif de l'invention, on obtient une avancée majeure dans le traitement de la douleur aiguë et chronique puisque tous les inconvénient sont résolus et les complications qui surviennent lors la forme traditionnelle visée au début. Les avantages sont très importantes par rapport a la simplicité du design, des avantages des coûts et du confort pour le patient ainsi que le gain en temps et le coût des matériaux.

### DESCRIPTION DES DESSINS

La figure 1.-Montre une vue du cathéter du dispositif de l'invention.
La figure 2.-Montre une vue de détail en vue de face et de côté de la pointe de aiguille du dispositif de l'invention.
La figure 3.-Montre une vue en coupe de l'aiguille du dispositif de l'invention.
La figure 4.-Montre une vue générale de l'aiguille du dispositif de l'invention.

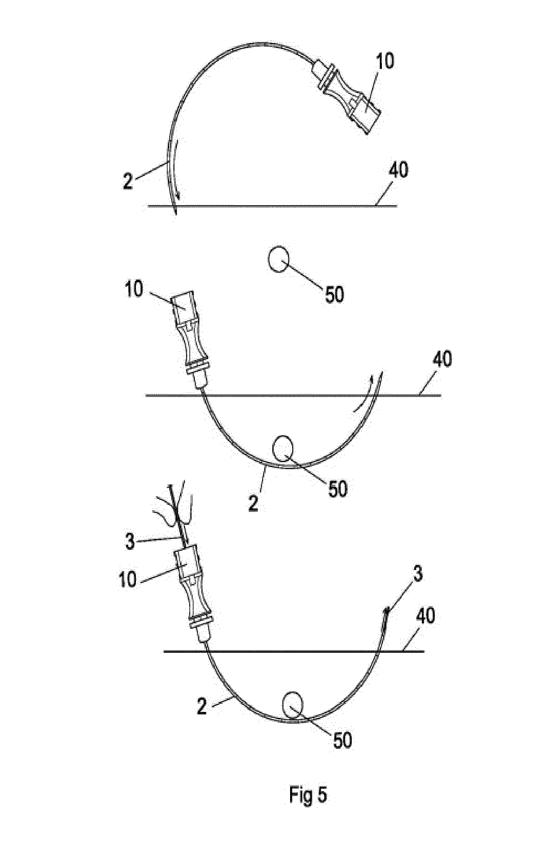

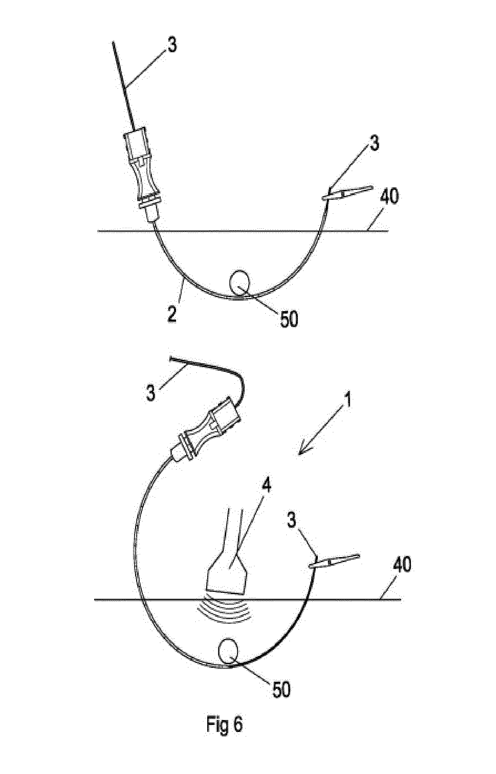

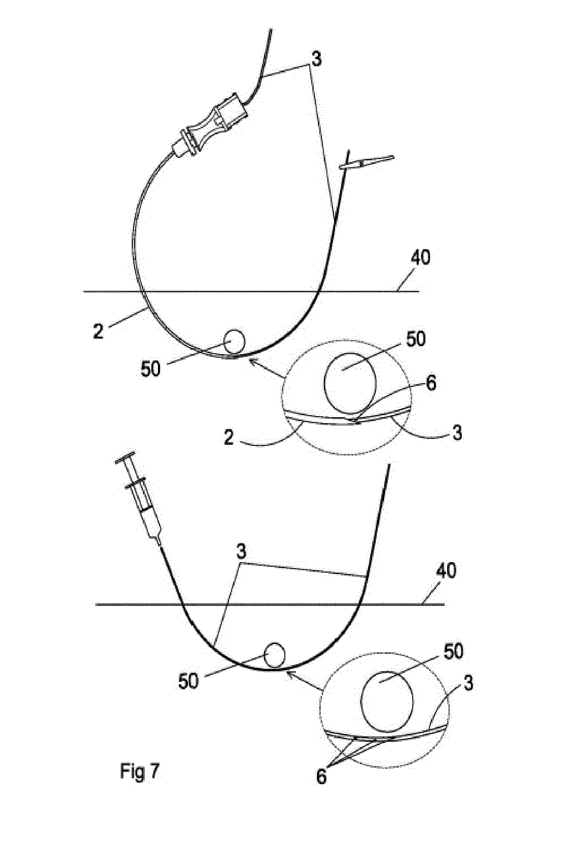

Les figures 5 à 7.- montrent une séquence d'utilisation du dispositif de l'invention, dans laquelle la figure 5 montre trois vue respectivement de: démarrage de l'introduction de l'aiguille, l'achèvement de l'introduction de l'aiguille dans les tissus du patient, et le cathéter est inséré dans l'aiguille; où la figure 6 montre deux vues respectivement de: maintenir la pointe du cathéter et le retrait de l'aiguille avec guidage écographique jusqu'à ce que sa pointe se trouve joint au nerf; et où la figure 7 montre deux vues respectivement de : l'insertion du cathéter jusqu'à ce que les ouvertures latéraux de la section tubulaire se trouvent joint au nerf et de l'application du médicament, après le retrait de l'aiguille.

### RÉALISATION PRÉFÉRENTIELLE de l'INVENTION

### MODE DE RÉALISATION PRÉFÉRÉ DE L'INVENTION

Le dispositif (1) pour positioner un cathéter (3) en un point d'application d'un médicament selon l'invention comprend une aiguille (2) creux et courbe d'un matériau détectable au moyen d'écographie (4), et un cathéter (3) formel et dimensionalmente apte à être introduit à l'inténieur de la dite aiguille (2), ayant besoin pour une performance optimale de la participation de l'écographie précitée (4) qui détecte la position de l'aiguille (2), comme illustre la figure 6. Le matériau de l'aiguille sera principalement l'acier inoxyrdable.

L'aiguille (2) peut incorporer extérieurement un marquage gradué (16) pour permettre le contrôle visuel direct sur la profondeur de aiguille et du nerf. Dans cet exemple de réalisation de l'invention ce marquage gradué (16) comprend quelques traits (17) de référence éloignés 1 centimètre entre eux. Cette graduation couvre idéalement toute la longueur de l'aiguille (2).

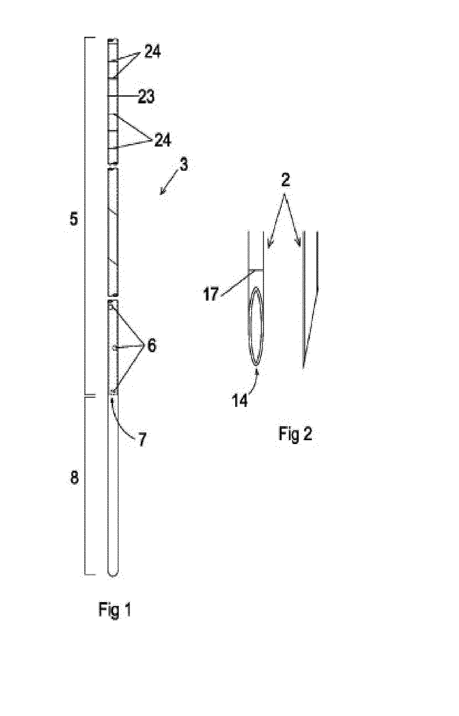

Le cathéter (3) (voir figure 1) comprend une section tubulaire (5) pour l'introduction du médicament, munie d ouvertures latérales (6) et achevé en une extrémité fermée (7), ainsi qu'un prolongement filiforme (8) qui permet la manipulation pendant son déploiement, et aussi quelques références de position de l'aiguille (2).

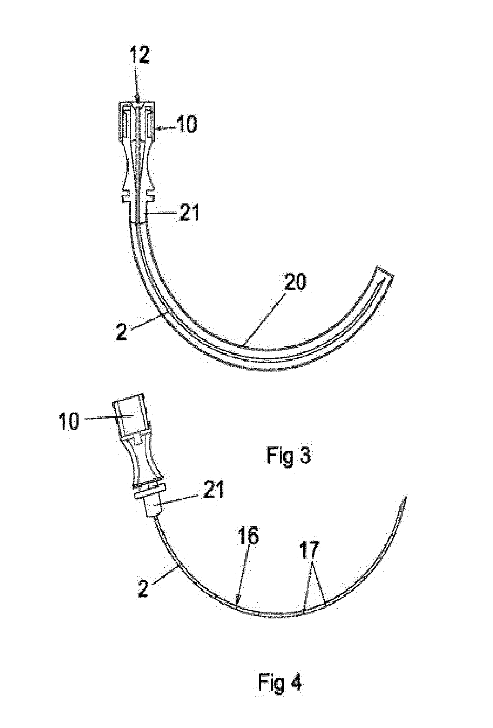

L'aiguille (2) (voir figures 2 à 4) comprend une poignée (10) anatomique de manipulation de son insertion, qui s'effectue en pénétrant dans les tissus (40) du patient, et rapproche la zone souhaitée pour l'application des médicaments, par exemple un nerf (50) comme on le voit dans les figures 5 à 7, revenant à sortir en dehors des tissus (40) du patient par sa forme incurvée. Une fois ainsi positionnée, le cathéter (3) est inséré par l'aiguille, comme on le voit dans la vue inférieure de la figure 5, pour laquelle l'aiguille (2) comprend idéalement un logement d'alimentation (12) du cathéter (3) à son entrée, le logement d'alimentation étant dans cet exemple de l'invention non limitatif (12), materéalisée dans la poignée (10) elle-même. En outre, l'aiguille (2) peut comprendre un capot de protection durable (20) dans une douille (21) du poignée (10) (voir figures 3 et 4).

La pointe de l'aiguille (2) a son extrémité émoussée (14), (voir un détail de forme figure 2) de sorte qu'il est possible d'avancer à travers les tissus (40), mais sans endommager le nerf (50) en cas d'un contact accidentel.

Pour sa part, la partie tubulaire (5) du cathéter (3) comprend trois ouvertures latérales (6), dont la première est adjacente à l'extrémité fermé (7) de la dite partie tubulaire (5) et les autres éloignées entre elles consécutivement du précédent dans le sens axiale principal du cathéter (3); se trouvent ces trois ouvertures laterales (6) disposées radicalement et en opposition de phase de 120 degrés, et étant l'espacemente dans la direction axiale du cathéter,(3), de préférence trois millimètres.

Dans cet exemple de réalisation, la prolongation filiforme (8) du cathéter est solide, et a une longueur d'au moins 14 centimètres, alors que les références de position comprennent marques visibles (23, 24) qui se trouvent réalisées à l'extérieur du cathéter (3) (voir figure 1), comprenant idéalement une première marque visible (23) et différenciée du reste des autres marques, située à une distance d'extrémité fermé (7) pour la partie tubulaire (5) du cathéter (3) équivalent à la longueur de l'aiguille (2) y compris la poignée, et des marques visibles supplémentaires (24) interéloignées depuis la première marque visible et entre elles, étant idéalement la distance entre les marques visibles (23, 24) d'un centimètre.

Le cathéter (3) est de préférence constitué dun matériau détectable par l'écographe, tel que du polyuréthane ou tout autre matièraux au l'avenir parmettrait d'ameliorer son guidage ecographique, toujours permettant maintenir la conception.

À la fois, l'aiguille (2) et le cathéter (3) peuvent être fabriqués en différentes tailles, citées à titre d'exemple non limitatif, un ensemble d'aiguille (2) de diamètre intérieur de calibre 17, 18 ou 19 gauges respectivement associé avec un cathéter (3) de diamètre calibre 18, 19 ou 20 gauges, avec des longueurs de l'aiguille comprises entre 90 et 150 milimetres et du cathéter entre 400 et 500 millimètres.

Avoir décrit suffisamentt la nature de l'invention, indique que la description de celle-ci et de son mode de réalisation préféré ne devrait pas être interprétée comme limitant, et qui couvre toutes les variantes possibles de réalisation qui suit inmédiatement le contenu d'ici et ses revendications.

## Revendications

1. Dispositif (1) pour placer un cathéter (3), dans un point d'application d'un médicament aves le contrôle direct de l'écographie, **caracterisé parce qu'il** se compose d'une aiguille (2) creuse courbe faite avec une matière détectable par l'écographie (4) et d'un cathéter (3) formelle et dimensionnelle adapte pour être introduit à l'intérieur de cette aiguille (2); où le cathéter (3) comprend une section tubulaire (5) pour l'introduction du médicament, munie de quelque ouvertures latérales (6) et achevé d'une extrémité fermée (7), une prolongation filiforme (8) de manipulation, et des références de position par rapport à l'aiguille (2).

2. Dispositif (1) pour placer un cathéter (3), dans un point d'application d'un médicament avec le contrôle direct de l'écographie selon revendication 1 **caractérisé parce que** l'aiguille (2) comprend une poignée (10) de manipulation pendant l'insertion anatomique

3. Dispositif (1) pour placer un cathéter (3) dans un point d'application d'un médicament avec contrôle direct de l'écographie selon une quelconque des revendications précédentes **caractérisé parce ce** que l'aiguille (2) a dans son entrée un logement d'alimentation(12) pour le cathéter (3).

4. Dispositif (1) pour placer un cathéter (3) dans un point d'application d'un médicament avec contrôle direct de l'écographie selon revendications 3 **caracterisé parce que** le logement d'alimentation (12) est matérialisé dans le poignée (10).

5. Dispositif (1) pour placer un cathéter (3) dans un point d'application d'un médicament avec contrôle direct de l'écographie selon l'une quelconque des revendications 2 à 4, **caracterisé parce que** l'aiguille (2) a un couvercle de protection (20) qui s'ajuste dans une douille (21) du poignée (10).

6. Dispositif (1) pour placer un cathéter (3) dans un point d'application d'un médicament avec contrôle direct de l'écographie selon l'une quelconque des revendications précédentes **caractérisé parce que** la pointe de l'aiguille (2) a son extrémité (14) emoussée.

7. Dispositif (1) pour placer un cathéter dans un point d'application d'un médicament avec contrôle direct de l 'écographie selon une quelconque des revendications précédentes **caractérisé parce que** l'aiguille (2) comprend un marquage gradué (16).

8. Dispositif (1) pour placer un cathéter (3) dans un point d'application d'un médicament avec contrôle direct de l'écographie selon revendication 7 **caractérisé parce que** le marquage gradué (16) couvre toute la longueur de l'aiguille (2).

9. Dispositif (1) pour placer un cathéter (3) dans un point d'application d'un médicament avec contrôle direct de l'écographie selon revendications 7 ou 8 **caractérisé parce que** le marquage gradué (16) comprend des lignes (17) espacées entre elles d'un centimètre.

10. Dispositif (1) pour placer un cathéter (3) dans un point d'application d'un médicament avec contrôle direct de l'écographie selon une quelconque des revendications précédentes **caractérisé parce que** la partie tubulaire (5) du cathéter (3) comprend trois ouvertures latérales (6), dont la première d'entre elles étant adjacente à l'extrémité fermée (7) de la dite partie tubulaire (5) et les autres consécutivement de la précédente dans le sens axiale principal du cathéter (3); disposées ces trois ouvertures latérales (6) radialement et en opposition de phase de 120 degrés.

11. Dispositif (1) pour placer un cathéter (3) dans un point d'application d'un médicament avec contrôle direct de l'écographie selon revendications 10 **caractérisé parce que** la distance des ouvertures latérales (6) dans le sens axiale principal du cathéter (3) est de trois millimètres.

12. Dispositif (1) pour placer un cathéter (3) dans un point d'application d'un médicament avec contrôle direct de l'écographie selon une quelconque des revendication précédentes **caractérisé parce que** la prolongation filiforme (8) est solide.

13. Dispositif (1) pour placer un cathéter (3) dans un point d'application d'un médicament avec contrôle direct de l'écographie selon une quelconque des revendications antérieurs **caractérisé parce que** la prolongation filiforme (8) vise une longueur d'au moins 14 centimètres.

14. Dispositif (1) pour placer un cathéter (3) dans un point d'application d'un médicament avec contrôle direct de l'écographie selon une quelconque des revendications précédentes **caractérisé parce que** les références de position au sujet de l'aiguille mise en oeuvre sur le cathéter (3) comprennent des marques visibles (23, 24) qui sont faites à l'extérieur du cathéter (3).

15. Dispositif (1) pour placer un cathéter (3) dans un point d'application d'un médicament avec contrôle direct de l'écographie selon revendications 14 **caractérisé parce qu**'il a une première marque visible (23) et différenciée du reste des autres marques, située à une distance d'extrémité fermée (7) de la partie tubulaire (5) du cathéter (3) équivalent à la longueur totale de l'aiguille, y compris la poignée (2), et des marques visibles supplémentaires (24) espacées depuis la première marque visible (23) et entre elles.

16. dispositif (1) pour placer un cathéter (3) dans un point d'application d'un médicament avec contrôle direct de l'écographie selon revendication 15 **caractérisé parce que** la distance entre les marques visibles (23, 24) est d'un centimètre.

17. Dispositif (1) pour placer un cathéter (3) dans un point d'application d'un médicament avec contrôle direct de l'écographie selon une quelconque des revendications précédentes **caractérisé parce que** l'aiguille (2) est matérialisée en acier inoxydable.

18. Dispositif (1) pour placer un cathéter (3) dans un point d'application d'un médicament avec contrôle direct de l'écographie selon une quelconque des revendication précédentes **caractérisé parce qu**'en outre le cathéter (3) est produit avec une matière détectable par l'écographie.

19. Dispositif (1) pour placer un cathéter (3) dans un point d'application d'un médicament avec contrôle direct de l'écographe selon revendication 18 **caractérisé parce que** le cathéter (3) est matérialisé en polyuréthane.
